# EUROPEAN PATENT APPLICATION

(11) **EP 1 900 733 A1**
(43) Date of publication of application: **19.03.2008**
(21) Application number: 06747292.8
(22) Date of filing: 07.06.2006
(51) Int. Cl.: C07D 401/12

(54) **PROCESS FOR PRODUCTION OF 4(3H)-QUINAZOLINONE DERIVATIVE**

(30) Priority: 07.06.2005 JP 2005167014
(71) Applicant: BANYU PHARMACEUTICAL CO., LTD., Tokyo 102-8667 (JP)
(72) Inventor: NAGASE, Tsuyoshi, Institute, 3, Okubo, Tsukuba-shi, Ibaraki 300-2611 (JP); SATO, Nagaaki, Institute, 3, Okubo, Tsukuba-shi, Ibaraki 300-2611 (JP); KII, Satoshi, 3-9-1, Kamimutsuna, Okazaki-shi, Aichi 444-0858 (JP); SATO, Kimihiko, 3-9-1, Kamimutsuna, Okazaki-shi, Aichi 444-0858 (JP); TSURITANI, Takayuki, 3-9-1, Kamimutsuna, Okazaki-shi, Aichi 444-0858 (JP); SAWADA, Naotaka, 3-9-1, Kamimutsuna, Okazaki-shi, Aichi 444-0858 (JP)
(74) Representative: Buchan, Gavin MacNicol
(86) International application number: PCT/JP2006/311886
(87) International publication number: WO 2006/132424

(57) **Abstract**

The present invention provides a process for producing a 4(3H)-quinazolinone derivative, which is useful as a medicinal substance, with better efficiency in an industrial scale. The process comprises the steps of reacting 4-hydroxy-N-tert-butoxycarbonylpiperidine with 4-fluoro-1-nitrobenzene in the presence of sodium hydride, reacting the resulting product with cyclobutanone, reducing the resulting product to give 4-(1-cyclobutyl-4-piperidinyl)oxyaniline, and reacting this compound with 2-methyl-5-trifluoromethyl-4H-3,1-benzoxazin-4-one to give 3-{4-[{1-cyclobutyl-4-piperidinyl)oxy]phenyl}-2-methyl-5-trifluoromethyl-4(3H)-quinazolinone.

## Description

### TECHNICAL FIELD

The present invention relates to a novel and more efficient process for producing a 4(3H)-quinazolinone derivative which is useful as a medicinal substance.

### BACKGROUND ART

A 4(3H)-quinazolinone derivative is useful as a preventive or a remedy for metabolic system diseases such as obesity, diabetes, hormone secretion disorder, hyperlipemia, gout, fatty liver; circulatory system diseases such as stenocardia acute/congestive heart failure, myocardial infarction, coronary arteriosclerosis, hypertension, renal disease, electrolyte disorder; and central and peripheral nervous system diseases such as bulimia, emotional disorder, depression, anxiety, delirium, dementia, schizophrenia, attention deficit hyperactivity disorder, memory impairment, Alzheimer disease, Parkinson disease, sleep disorder, cognitive disorder, motion disorder, paresthesia, dysosmia, epilepsy, morphine resistance, narcotic dependence, alcoholism (Patent Reference 1).
Patent Reference 1 discloses a process for producing a 4(3H)-quinazolinone derivative.
Patent Reference 1: WO2005/077905

### DISCLOSURE OF THE INVENTION

The process for producing a 4(3H)-quinazolinone derivative disclosed in Patent Reference 1 needs improvements for industrial-scale production of the derivative in that, for example, it includes a step having a low yield.

The present inventors have assiduously studied the process for producing a 4(3H)-quinazolinone derivative disclosed in Patent Reference 1, for the purpose of developing a process for producing a 4(3H)-quinazolinone derivative that is efficient as an industrial-scale production process, and have completed the present invention.

Specifically, the invention relates to the following (a) to (g):
(a) A process for producing 3-{4-[(1-cyclobutyl-4-piperidinyl)oxy]phenyl}-2-methyl-5-trifluoromethyl-4(3H)-quinazolinone, which comprises reacting 2-methyl-5-trifluoromethyl-4H-3,1-benzoxazin-4-one with 4-(1-cyclobutyl-4-piperidinyl)oxyaniline or its acid-addition salt in the presence of an acid.
(b) The production process of (a), wherein the acid is acetic acid.
(c) The production process of (b), wherein sodium acetate is further added to the reaction system.
(d) The production process of (a), wherein 4-(1-cyclobutyl-4-piperidinyl)oxyaniline or its acid-addition salt is prepared according to the following:
   (1) a step of reacting 4-hydroxy-N-tert-butoxycarbonylpiperidine with 4-fluoro-1-nitrobenzene in the presence of a base to produce 4-(4-piperidinyl)oxy-N-tert-butoxycarbonyl-1-nitrobenzene,
   (2) a step of deprotecting 4-(4-piperidinyl)oxy-N-tert-butoxycarbonyl-1-nitrobenzene to produce 4-(4-piperidinyl)oxy-1-nitrobenzene,
   (3) a step of reacting 4-(4-piperidinyl)oxy-1-nitrobenzene with cyclobutanone in an inert solvent to produce 4-(1-cyclobutyl-4-piperidinyl)oxy-1-nitrobenzene, and
   (4) a step of reducing 4-(1-cyclobutyl-4-piperidinyl)oxy-1-nitrobenzene in an inert solvent.
(e) The production process of (a), wherein 4-(1-cyclobutyl-4-piperidinyl)oxyaniline or its acid-addition salt is prepared according to the following:
   (1) a step of reacting 4-hydroxypiperidine with cyclobutanone in an inert solvent to produce 1-cyclobutyl-4-hydroxypiperidine,
   (2) a step of reacting 1-cyclobutyl-4-hydroxypiperidine with 4-fluoro-1-nitrobenzene in the presence of a base to produce 1-cyclobutyl-4-(4-nitro-phenoxy)-piperidine, and
   (3) a step of reducing 1-cyclobutyl-4-(4-nitro-phenoxy)-piperidine in an inert solvent.
(f) The production process of (a), wherein 2-methyl-5-trifluoromethyl-4H-3,1-benzoxazin-4-one is prepared by reacting 2-amino-6-trifluoromethylbenzoic acid or its salt with acetic anhydride in an inert solvent.
(g) The production process of (a), wherein the acid-addition salt of 4-(1-cyclobutyl-4-piperidinyl)oxyaniline is 4-(1-cyclobutyl-4-piperidinyl)oxyaniline p-toluenesulfonate.

### BEST MODE FOR CARRYING OUT THE INVENTION

The method for producing a 4(3H)-quinazolinone derivative of the invention is described concretely.

### (Step A1)

The step of "reacting 4-hydroxy-N-tert-butoxycarbonylpiperidine with 4-fluoro-1-nitrobenzene in the presence of a base to produce 4-(4-piperidinyl)oxy-N-tert-butoxycarbonyl-1-nitrobenzene" may be carried out as follows: 4-Hydroxy-N-tert-butoxycarbonylpiperidine is dissolved in an inert solvent such as dimethylformaldehyde (DMF) or tetrahydrofuran, then a base is gradually added thereto at 0°C or lower in a nitrogen atmosphere, and reacted at room temperature for 0.5 to 10 hours, preferably for 0.5 to 2 hours; then a 4-fluoro-1-nitrobenzene/dimethylformaldehyde solution is dropwise added to the resulting reaction solution preferably at 0°C, and then reacted at 0°C to 50°C, preferably at room temperature to 30°C for 1 to 24 hours, preferably for 10 to 24 hours.

Regarding the amount of 4-hydroxy-N-tert-butoxycarbonylpiperidine, the base and 4-fluoro-1-nitrobenzene to be used, it is recommended that the amount of the base is from an equimolar amount to 5 mols, preferably from 1.2 to 2 mols relative to one mol of 4-hydroxy-N-tert-butoxycarbonylpiperidine, and that the amount of 4-fluoro-1-nitrobenzene is from an equimolar amount to 5 mols, preferably from an equimolar amount to 2 mols.

Examples of the base are potassium t-butoxide, sodium hydride, n-butyllithium, lithium diisopropylamide (LDA), lithium hexamethyldisilazane (LIHMDS), potassium t-butoxide, sodium hexamethyldisilazane (NaHMDS), isopropylmagnesium chloride (iPrMgCl) et al.

When combined with a phase transfer catalyst, other bases such as sodium hydroxide or potassium hydroxides are also usable in addition to the above-mentioned bases. Examples of the phase transfer catalyst are benzyltetraethylammonium chloride, tetrabutylammonium bromide et al. The amount of the phase transfer catalyst to be used is recommendably from 0.01 to 0.1 equivalents to the base, preferably from 0.05 to 0.08 equivalents.

### (Step A2)

The step of "deprotecting 4-(4-piperidinyl)oxy-N-tert-butoxycarbonyl-1-nitrobenzene to produce 4-(4-piperidinyl)oxy-1-nitrobenzene" may be carried out as follows: 4-(4-Piperidinyl)oxy-N-tert-butoxycarbonyl-1-nitrobenzene is added to, for example, trifluoroacetic acid, and reacted at 0°C to 50°C, preferably at 0°C to room temperature for 1 to 10 hours, preferably for 1 to 2 hours.

The deprotection may be attained by the use of trifluoroacetic acid. The amount of trifluoroacetic acid to be used in the case is recommendably from I to 10 mL relative to 1 g of 4-(4-piperidinyl)oxy-N-tert-butoxycarbonyl-1-nitrobenzene, preferably from I to 5 mL.

In addition to the above, the methods described in Protective Groups in Organic Synthesis by T. W. Green, 2nd Ed., John Wiley & Sons, 1991 are also applicable to the deprotection of the tert-butoxycarbonyl group.

### (Step A3)

The step of "reacting 4-(4-piperidinyl)oxy-1-nitrobenzene with cyclobutanone in an inert solvent to produce 4-(1-cyclobutyl-4-piperidinyl)oxy-1-nitrobenzene" may be carried out as follows: 4-(4-Piperidinyl)oxy-1-nitrobenzene, cyclobutanone and optionally an acid are dissolved in a protic solvent such as methanol, ethanol or propanol, or in an mert solvent such as dichloromethane, chloroform, tetrahydrofuran or diethyl ether, then a reducing agent is added thereto at room temperature, and reacted at room temperature for 1 to 20 hours, preferably for 3 to 10 hours.

The acid to be used in this step includes, for example, acetic acid, ZnCl₂ et al.

The reducing agent includes sodium borohydride, sodium cyanoborohydride, sodium triacetoxyborohydride et al.

Regarding the amount of 4-(4-piperidinyl)oxy-1-nitrobenzene, cyclobutanone, the acid and the reducing agent to be used, the amount of the acid is recommendably from 0.! to 3 mols, preferably from 0.1 to 1.5 mols relative to one mol of 4-(4-piperidinyl)oxy-1-nitrobenzene, and the amount of cyclobutanone is recommendably from 1 to 5 mols, preferably from 1.0 to 2.0 mols, the amount of the reducing agent is recommendably from 1.0 to 2.0 mols.

### (Step A4)

The step of "reducing 4-(1-cyclobutyl-4-piperidinyl)oxy-1-nitrobenzene in an inert solvent" may be carried out as follows: 4-(1-Cyclobutyl-4-piperidinyl)oxy-1-nitrobenzene is dissolved in a protic solvent such as methanol or ethanol, or any other organic solvent generally usable in catalytic hydrogen reduction, then for example, palladium-carbon (Pd-C) is added thereto in a nitrogen atmosphere, and the gas in the reactor is purged away, and hydrogen gas is introduced thereinto under forcedly stirring, and reacted at room temperature for 1 to 30 hours, preferably for 10 to 15 hours. The hydrogen pressure is, for example, 1 to 4 atmospheric pressures.

The amount of palladium-carbon to be used is, for example, recommendably from 0.01 to 1 g relative to 1g of 4-(1-cydohutyl-4-piperidinyl)oxy-1-nitrobenzene, preferably from 0.1 to 0.5 g in the case of 10 % palladium-carbon.

As the hydrogen source, also usable is formic acid in place of hydrogen gas. In this case, the amount of formic acid to be used is, for example, recommendably from I to 10 equivalents relative to one equivalent of 4-(1-cyclobutyl-4-piperidinyl)oxy-1-nitrobenzene, preferably from 2 to 5 equivalents.

A Raney nickel or the like which can be used in ordinary catalytic hydrogen reduction as a catalyst may also be used, in addition to palladium-carbon. Further, in addition to the reduction method of catalytic hydrogen reduction, also employable herein is any other reducing agent of, for example, lithiumaluminium hydride, isobutylaluminium hydride or a combination of metal salts such as sodium borohydride/nickel chloride.

The acid-addition salt of 4-(1-cyclobutyl-4-piperidinyl)oxyaniline includes p-toluenesulfonate thereof, and the salt may be prepared by dissolving 4-(1-cyclobutyl-4-piperidinyl)oxyaniline in ethyl acetate, then adding a ethanol solution of p-toluenesulfonic acid monohydrate thereto, heating until the deposit could be dissolved, and thereafter leaving it at room temperature.

The amount of p-toluenesulfonic acid monohydrate to be used is recommendably an equimolar amount to that of 4-(1-cyclobutyl-4-piperidinyl)oxyaniline.

Dunng the reduction in the step 4A, p-toluenesulfonic acid monohydrate may be added to prepare 4-(1-cyclobutyl-4-piperidinyl)oxyaniline p-toluenesulfonate simultaneously with the reduction.

### (Step A4-2)

The step of "producing 2-methyl-5-(trifluoromethyl)-4H-3,1-benzoxazin-4-one by reacting 2-amino-6-trifluoromethylbenzoic acid or its acid-addition salt with acetic anhydride in an inert solvent" may be carried out as follows, 2-Amino-6-trifluoromethylbenzoic acid or its salt is suspended in tetrahydrofuran, then acetic anhydride is added thereto, and refluxed for 1 to 20 hours, preferably for 5 to 10 hours.

The amount of acetic anhydride to be used is recommendably from 1.0 to 20 mols relative to 1 mol of 2-amino-6-trifluoromethylbenzoic acid, preferably from 4.0 to 10.0 mols.

The salt of 2-amino-6-trifluoromethylbenzoic acid includes, for example, hydrochloride, hydrobromide, sulfate, trifluoroacetate, p-toluenesulfonate and phosphate thereof.

### (Step A5)

The step of "reacting 2-methyl-5-trifluoromethyl-4H-3,1-benzoxazin-4-one with 4-(1-cyclobutyl-4-piperidinyl)oxyaniline or its acid-addition salt in the presence of an acid" may be carried out by reacting the two in the presence of an acid, in an inert solvent or a mixed solvent thereof or in the absence of a solvent.

The reaction temperature is for example, recommendably from room temperature to 150°C, preferably from room temperature to 50°C; and the reaction time is recommendably from 1 to 50 hours, preferably from 10 to 30 hours.

Examples of the inert solvent are toluene, benzene, hexane, diethyl ether, tetrahydrofuran, 1,2-dimethoxymethane et al.

The amount of 4-(1-cyclobutyl-4-piperidinyl)oxyaniline or its acid-addition salt to be used is, for example, recommendably from an equimolar amount to 5 mols relative to 1 mol of 2-methyl-5-trifluoromethyl-4H-3,1-benzoxazin-4-one, preferably from an equimolar amount to 1.5 mols.

The acid to be used is preferably acetic acid, and the amount to be used is recommendably from 0.1 equivalent to a large excessive amount relative to 2-methyl-5-trifluoromethyl-4H-3,1-benzoxazin-4-one, preferably from 5 to 30 times by volume of 2-methyl-5-trifluoromethyl-4H-3,1-benzoxazin-4-one. In case acetic acid serves as a solvent, an additional solvent may be omitted.

On the other hand, sodium acetate may also be added to the reaction system. The amount to be used in this case is, for example, recommendably from an equimolar amount to 5 molar times relative to 2-methyl-5-trifluoromethyl-4H-3,1-benzoxazin-4-one, preferably from an equimolar amount to 1.2 molar times, more preferably an equimolar amount.

In particular, as a combination of acetic acid, sodium acetate and solvent, the following are recommended:
(a) Acetic acid of from 10 to 30 times by volume of 2-methyl-5-trifluoromethyl-4H-3,1-benzoxazin-4-one;
(b) Acetic acid of 10 times by volume of 2-methyl-5-trifluoromethyl-1H-3,1-benzoxazin-4-one, and sodium acetate of an equimolar amount to 2-methyl-5-trifluoromethyl-4H-3,1-benzoxazin-4-one;
(c) Acetic acid of 10 times by volume and tetrahydrofuran of 10 times by volume of 2-methyl-5-trifluoromethyl-4H-3,1-benzoxazin-4-one and sodium acetate of from an equimolar amount to 5 molar times relative to 2-methyl-5-trifluoromethyl-4H-3,1-benzoxazin-4-one;
(d) Acetic acid of 10 times by volume and toluene of 20 times by volume of 2-methyl-5-trifluoromethyl-4H-3,1-benzoxazin-4-one, and sodium acetate of from an equimolar amount to 5 molar times relative to 2-methyl-5-trifluoromethyl-4H-3,1-benzoxazin-4-one.

4-(1-Cyclobutyl-4-piperidinyl)oxyaniline may also be prepared according to a process comprising the steps mentioned below.

### (Step B1)

A step of reacting 4-hydroxypiperidine with cyclobutanone in an inert solvent to produce 1-cyclobutyl-4-hydroxypiperidine.

### (Step B2)

A step of reacting 1-cyclobutyl-4-hydroxypiperidine with 4-fluoro-1-nitrobenzene in the presence of a base to produce 1-cyclobutyl-4-(4-nitro-phenoxy)-piperidine.

### (Step B3)

A step of reducing 1-cyclobutyl-4-(4-nitro-phenoxy)-piperidine in an inert solvent to give 4-(1-cyclobutyl-4-piperidinyl)oxyaniline.

The reaction of the step B1 may be carried out in accordance with the step A3. In this step, the acid used in the step A3 may be optionally added.

The reaction of the step B2 may be carried out in accordance with the step A1. Especially in this step, combined use of a base and a phase transfer catalyst is recommended.

The reaction of the step B3 may be carried out in accordance with the step A4. In this step, it is recommended to use palladium-carbon as the catalyst in catalytic hydrogen reduction, and to use formic acid as the hydrogen source.

The above-mentioned compounds thus obtained in the manner as above may be isolated and purified in any known separation and purification method, for example, concentration, concentration under reduced pressure, solvent extraction, crystallization, recrystallization, reprecipitation, chromatography et al.

### Examples:

The invention is described concretely with reference to the following Examples, to which, however, the invention should not be limited.

As the plate in thin-layer chromatography in Examples, used was Silica gel 60F245 (Merek); and for detection, used was a UV detector. As the column silica gel, used was Wakogel^{™} C-300 (Wako Pure Chemical Industries); and as the reversed-phase column silica gel, used was LC-SORBTM SP-B-ODS (Chemco) or YMC-GELTM ODS-AQ 120-S50 (Yamamura Chemical Laboratories). The mass spectrum was measured with Quattroll (Micromass), according an electrospray ionization (ESI) method. The melting point was measured, using a differential scanning calorimeter, Q1000 (TA Instrument Japan). The NMR spectrum was measured, using dimethyl sulfoxide as the internal standard in a heavy dimethyl sulfoxide solution. For this, used were ADVANCE300 (300 MHz; BRUKER), ADVANCE500 (500 MHz; BRUKER), Mercury400 (400 MHz; Varian) and Inova400 (400 MHz: Varian)-type spectrometers; and all δ data were expressed as ppm.
CDCl₃: heavy chloroform,
CD₃OD: heavy methanol,
DMSO-d₀: heavy dimethyl sulfoxide,

The meanings of the abbreviations in nuclear magnetic resonance spectrometry arc shown below.
s: singlet,
d: doublet,
dd: double doublet,
t: triplet.
m; multiplet,
br: broad.
q: quartet,
J: coupling constant.
Hz; hertz.
Example 1-1:

### Production of 4-(4-piperidinyl)oxy-N-tert-butoxycarbonyl-1-nitrobenzene

In a nitrogen atmosphere, a dimethylformamide solution (50 mL) of N-tert-butoxycarbonyl-4-piperidinol (10.0 g, 49.7 mmol) was cooled with ice, and sodium hydride (3.0 g, 75 mmol. as 60 % content) was added thereto little by little, and stirred at room temperature for 30 minutes. The reaction mixture was again cooled in an ice bath, and a dimethylformamide solution (10 mL) of 4-fluoronitrobenzene (7.71 g, 55 mmol) was dropwise added thereto, and stirred at room temperature for 15 hours. Water (1 5 mL) was added to it to stop the reaction, and the solvent was evaporated off at 50°C under reduced pressure. Water (120 mL) was added to the residue, extracted with ethyl acetate (120 mL x 2), and the organic layer was washed twice with distilled water, and dried with anhydrous sodium sulfate. After filtered, the filtrate was concentrated under reduced pressure, and the resulting yellow solid was washed in a mixed solvent of isopropyl ether/hexane (1/4) under stirring. The solid was taken out through filtration, and dried under reduced pressure to obtain 4-(4-piperidinyl)oxy-N-tert-butoxycarbonyl-1-nitrobenzene (12.4 g, 77 %) as a pale yellow solid.
ESI m/z: 345.2 [M +Na]⁺
¹H-NMR (400 MHz, CDCl₃, δ ppm): 1.48 (9H, s), 1.75-1.83 (2H, m), 1.93-2.00 (2H, m), 3.35-3.42 (2H, m), 3.67-3.73 (2H, m), 4.58-4.63 (1H, m), 6.96 (2H, d, J=9.3 Hz), 8.20 (2H, d. J-9.3 Hz).

### Example 1-2:

### Production of 4-(4-piperidinyl)oxy-1-nitrobenzene

4-(4-Piperidinyl)oxy-N-tert-butoxycarbonyl-1-nitrobenzene (10.9 g. 33.8 mmol) was dissolved in trifluoroacetic acid (30 mL), and stirred at room temperature for 1 hour. The excessive trifluoroacetic acid was evaporated off under reduced pressure, and ethyl acetate (150 mL) and aqueous 2 N sodium hydroxide solution (100 mL) were added to the residue. (In this stage, the aqueous layer was confirmed to have a pH of at least 9.) The resulting mixture was extracted twice with ethyl acetate, and the organic layer was washed with aqueous 1 N sodium hydroxide solution and saturated saline in that order, and dried with anhydrous sodium sulfate. After filtered, the organic layer was concentrated, and the residue was dried under reduced pressure to obtain 4-(4-piperidinyl)oxy-1-nitrobenzene (7.5 g, quantitative) as a brown oil.
ESI m/z: 223.1 [M+H]⁺
¹H-NMR (400 MHz, CDCl₃, δ ppm): 1.70-1.79 (2H, m), 2.03-2.09 (2H, m), 2.23 (1H, brs), 2.77-2.83 (2H, m), 3.14-3.20 (2H, m), 4.49-4.55 (1 H, m), 6.95 (2H, d, J-9.3 Hz), 8.19 (2H, d, J- 9.3 Hz).

### Example 1-3:

### Production of 4-(1-cyclobutyl-4-piperidinyl)oxy-1-nitrobenzene

4-(4-Piperidinyl)oxy-1-nitrobenzene (7.5 g, 33.8 mmol), zinc(II) chloride (4.61 g, 33.8 mmol) and cyclobutanone (3.55 g, 50.7 mmol) were suspended in methanol (100 mL), and sodium cyanoborohydride (3.19 g, 50.7 mmol) was added thereto and stirred at room temperature for 5 hours. The reaction mixture was concentrated under reduced pressure, then ethyl acetate and aqueous 2 N sodium hydroxide solution were added to the residue, and the resulting insoluble matter was removed through filtration with Celite. The filtrate was subjected to liquid-liquid separation, then the organic layer was washed with aqueous 1N sodium hydroxide solution and saturated saline in that order, and dried with anhydrous sodium sulfate. After filtered, the filtrate was concentrated, and the resulting solid was washed in a mixed solvent of isopropyl ether/hexane (1/4) under stirring. The solid was taken out through filtration, and dried under reduced pressure to obtain 4-(1-cyclobutyl-4-piperidinyl)oxy-1-nitrobenzene (7.5 g, 80 %) as a pale yellow solid.
ESI m/z: 277.3 [M+H]⁺
¹H-NMR (400 MHz, CDCl₃, δ ppm): 1.76-1.94 (2H, m), 2.06-2.16 (2H, m), 2.22-2.32 (6H, m), 2.90-3.12 (4H, m), 3.28-3.36 (1H, m), 4.76 (1H, brs), 6.99 (2H, d, J-9.2 Hz), 8.19 (2H, d, J-9.2 Hz).

### Example 1-4:

### Production of 4-(1-cyclobutyl-4-piperidinyl)oxyaniline p-toluenesulfonate

In a nitrogen atmosphere, 10 % palladium-carbon (3.0 g) was added to a methanol solution (100 mL) of 4-(1-cyclobutyl-4-piperidinyl)oxy-1-nitrobenze (7.5 g 27 mmol). The reactor was purged with hydrogen, and the mixture was vigorously stirred at room temperature for 13 hours. Palladium-carbon was removed through filtration with Celite, and the filtrate was concentrated. The residue was dried under reduced pressure to obtain 4-(1-cyclobutyl-4-piperidinyl)oxyaniline (6.5 g, 97 %) as a pale brown oil.
ESI m/z: 247.3 [M+H]⁺
¹H-NMR (400 MHz, CDl₃, δ ppm): 1.62-2.13 (12H, m), 2.62 (2H, brs), 2.68-2.76 (1H, m), 3.43 (2H, brs), 4.09-4.15 (1H, m), 6.62 (2H, d, J=8.8 Hz), 6.76 (2H, d, J=8.8 Hz).

An ethanol solution (20 mL) of p-toluenesulfonic acid monohydrate (5.02 g, 26.4 mmol) was added to an ethyl acetate solution (100 mL) of 4-(1-cyclobutyl-4-piperidinyl)oxyaniline (6.5 g. 26.4 mmol). The suspension was heated to completely dissolve, and then cooled to room temperature under slowly stirring. The precipitated solid was taken out through filtration, and the solid was washed with ethyl acetate. The resulting solid was dried under reduced pressure to obtain 4-(1-cyclobutyl-4-piperidinyl)oxyaniline p-toluenesulfonate (10.3 g, 93 %) as a pale brown solid.
ESI m/z: 247.3 [M+H]⁺
¹H-NMR (400 MHz, CDCl₃/CD₃OD-4/1, δ ppm); 1.68-1.79 (1H, m), 1.81-1.90 (1H, m), 2.09-2.15 (2H, m), 2.19-2.27 (2H, m), 2.34-2.43 (5H, m), 2.52-2.61 (2H, m), 2.86-2.93 (2H, m), 3.34-3.44 (3H, m), 4.52 (1H, brs), 6.70-6.75 (4H, m), 7.21 (2H, d, J=7.8 Hz), 7.79 (2H, d, J=7.8 Hz).

### Example 1-5:

### Production of 2-methyl-5-(trifluoromethyl)-4H-3,1-benzoxazin-4-one

2-Amino-6-trifluoromethylbenzoic acid trihydrate (12.0 g. 46.3 mmol) purchased from Apollo Scientific Ltd. was suspended in tetrahydrofuran (120 mL), and acetic anhydride (21.9 mL, 232 mmol) was added to the suspension and heated under reflux for 6 hours. The reaction liquid was filtered, and the filtrate was concentrated (until the total volume thereof could he about 24 mL). N-heptane (120 mL) was added to the residue, and stirred at room temperature for 1 hour. The formed solid was taken out through filtration, washed with n-heptane (24 mL), and dried at 60°C for 15 hours, to obtain the entitled compound (9.55 g, 90 %) as a colorless crystal.
ESI m/z: 230.2 [M+H]⁺
¹H-NMR (400 MHz, CDCl₃, δ ppm): 2.49 (3H, s), 7.76 (1H, dd, J=2.0, 7.3 Hz), 7.84-7.91 (2H, m).

### Example 1-6:

### Production of 3-[4-[(1-cyclobutyl-4-piperidinyl)oxy]phenyl}-2-methyl-5-trifluoromethyl-4(3H)-quinazolinone

The compound (11.0 g, 47.8 mmol) obtained in Example 1-5 was added to an acetic acid solution (110 mL) of 4-(1-cyclobutyl-4-piperidinyl)oxyaniline p-toluenesulfonate (20.0 g, 47.8 mmol), and stirred at room temperature for 18 hours. The solvent was evaporated off under reduced pressure, and ethyl acetate (400 mL) was added to the residue. The organic layer was washed with aqueous 2 N sodium hydroxide solution (200 mL) and saturated saline (100 mL) in that order, and dried with anhydrous sodium sulfate. After filtered, the filtrate was concentrated, and the residue was purified through silica gel column chromatography (Biotage Si 75 L, chloroform/methanol - 100/0 to 99/1 to 98/2), and recrystallized from hot ethyl acetate to obtain 3-{4-[(1-cyclobutyl-4-piperidinyl)oxy]phenyl}-2-methyl-5-trifluoromethyl-4(3H)-quinazolinone (13.8 g, 63 %) as a colorless crystal.
ESI m/z: 458.2 [M+H]⁺
¹H-NMR (400 MHz, CDCl₃, δ ppm): 1.65-1.76 (2H, m), 1.83-1.95 (411, m), 2.00-2.09 (4H, m), 2.15-2.25 (2H, m), 2.27 (3H, s), 2.59-2.65 (2H, m), 2.72-2.80 (1H, m). 4.35-4.40 (1H, m), 7.04 (2H, d, J=8.8 Hz), 7.15 (2H, d, J=8.8 Hz), 7.80 (1H, t, J=7.8 Hz), 7.86-7.89 (2H, m).

### Example 2:

### Another production of 3-{4-[(1-cyclobutyl-4-piperidinyl)oxy]phenyl}-2-methyl-5-trifluoromethyl-4(3H)-quinazolinone

### 1) Production of 1-cyclobutyl-4-hydroxypiperidine:

Tetrahydrofuran (20 L), 4-hydroxypiperidine (1.0 kg, 9.9 mol) and cyclobutanone (889 mL, 11.9 mol) were added to a 30-L vessel. The solution was cooled to 0°C, sand sodium triacetoxyborohydride (2.5 kg, 11.9 mol) was added thereto. The suspension was wanned up to room temperature (22 to 25°C). After stirred for 4 hours, the suspension was cooled to 0°C. Aqueous 5N sodium hydroxide solution (2.4 L) was added to the resulting reaction liquid to quench the reaction. Using 1 N hydrochloric acid (10 L), the organic layer was acidified, and then 20 % saline (4 L) was added thereto. The aqueous layer was made basic by adding aqueous 5N sodium hydroxide solution thereto. The tetrahydrofuran layer was separated from the mixture through liquid-liquid separation, and analyzed through 1H-NMR, which confirmed the presence of 308 g of the intended product in the mixture. (The calculated yield is 20 %. This is hereinafter referred to as assay yield). The separated liquid layer was made to have a pH of at least 14 by adding aqueous 5 N sodium hydroxide solution (5 L) thereto. Tetrahydrofuran (10 L) was added to the aqueous layer to recover 1,157 g of the intended product (75 % assay yield). The tetrahydrofuran layers were combined, and concentrated with a rotary evaporator to obtain 1.46 kg of the entitled compound as an orange oil (95 % assay yield).
¹H-NMR (500 MHz, DMSO-d₆, δ ppm): 1.34 (m, 2H), 1.60 (m, 2H), 1.69-1.83 (brm, 6H), 1.95 (m, 2H), 2.58-2.66 (brm, 3H), 3.44 (brs, 1H), 4.64 (brs, 1H).

### 2) Production of 1-cyclobutyl-4-(4-nitrophenoxy)-piperidine:

Toluene (11.6 L) and the above compound (1.45 kg as the pure content, 9.34 mol) were added to a 30-1 vessel. The solution was cooled to 0°C, and aqueous 8 N potassium hydroxide solution (5.1 L) and benzyltriethylammonium chloride (170.2 g) were added to the solution. Next, 4-fluoronitrobenzene (1.04 L, 9.8 mol) was dropwise added to the reaction liquid. The mixture was warmed up to room temperature (22 to 25°C), and stirred for 88 hours. The organic layer was separated through liquid-liquid separation, and 1 N hydrochloric acid (11.6 L) was added to the organic layer. After liquid-liquid separation, aqueous 5 N sodium hydroxide solution (8.7 L) was added to the aqueous layer to make it basic, and then tertiary butyl methyl ether (21.8 L) was added to it for extraction. The organic layer contained 1.57 kg of the entitled compound (61 % assay yield).
¹H-NMR (500 MHz, DMSO-d₆, δ ppm): 1.65 (m, 4H), 1.80 (m, 2H), 1.98 (m, 4H), 2.11 (t, J=9.5 Hz, 2H), 2.58 (brm, 2H), 2.73 (m, 1H), 4.61 (m, 1H), 7 18 (d, J-9.3 Hz, 2H), 8.21 (d, J=9.3 Hz, 2H).

### 3) Production of 4-(1-cyclobutyl-4-piperidinyl)oxyaniline p-toluenesulfonate:

The tertiary butyl methyl ether solution of the compound obtained in 2) was concentrated to 7.8 L. Methanol (15.6 L) was added to the concentrate. The solution was concentrated to 7.8 L, and 7.8 L of methanol was further added thereto, and thereafter the solution was further concentrated to 13.4 L. The concentrate was added to a 30-L vessel, and methanol (2.2 L) was added thereto for washing. Palladium-carbon (6 % by weight, 93.6 g) was added to it, and then formic acid (860 mL) was dropwise added thereto so that the inner temperature could be 35°C or lower, over 150 minutes. 10 minutes after the addition, the ratio of the starting compound, 1-cyulobutyl-4-(4-nitrophenoxy)-piperidine to the intended product was 0.5 to 98.7. Next, a methanol (4.76 L) solution of toluenesulfonic acid monohydrate (1.07 kg) was added in a nitrogen atmosphere. After stirred for 5 minutes, palladium-carbon was removed through filtration, and the resulting solution was transferred into a rotary evaporator. The solution was concentrated to 7.8 L, whereupon a crystal began to deposit. Isopropylacetic acid (15.6 L) was added dropwise to the slurry over 30 minutes. After stirred for 20 minutes, n-heptane (15.6 L) was dropwise added to it over 30 minutes, and then stirred overnight. The concentration of the entitled compound in the supernatant was 2.55 mg/mL. The crystal was taken out through filtration, then washed with n-heptane (1.56 L), and dried in a nitrogen atmosphere for 2.5 hours. The mother liquid and the wash liquid contained the intended product in an amount of 72.3 g (3 % assay yield) and 1.8 g (< 1 % assay yield), respectively. The crystal was further dried overnight under reduced pressure at 50°C to obtain 2.15 kg of the entitled compound (91 % yield).
¹H-NMR (500 MHz, CDCl₃, δ ppm): 1.63-1.79 (m, 1H), 1,79-1.93 (m, 2H), 2.04-2.28 (m, 5H), 2.36 (s, 3H), 2.42-2.78 (m, 5H), 2.80-2.95 (m, 2H), 4.51 (bs, 1H), 6.66 (d, J=8.9 Hz, 2H), 6.71 (d, J=8,9 Hz, 2H), 7.19 (d, J=8.0 Hz. 2H), 7.82 (d, J=8.0 Hz, 2H).

### 4) Production of 3-{4-[(1-cyclobutyl-4-piperidinyl)oxy]phenyl}-2-methyl-5-trifluoromethyl-4(3H)-quinazolinone:

Tetrahydrofuran (3.6 L), the compound obtained in 3) (1.81 kg. 4.32 mol), 2-methyl-5-trifluoromethyl-4H-3,1-benzoxazin-4-one (899.9 g, 3.93 mol) and sodium acetate (386.9 g, 4.71 mol) were added to a 50-L vessel. Further, toluene (18.0 L) and acetic acid (2.36 kg, 39.27 mol) were added to it, and warmed up to 40°C over 3.5 hours, and then stirred for 41 hours. The end point of the reaction was monitored by high-performance liquid chromatography, and then the reaction was quenched with a mixture of water (15.0 L) and aqueous 48 % sodium hydroxide solution (4.52 kg). After liquid-liquid separation, a mixture of toluene and tetrahydrofuran (4.5 L and 0.9 L) was added to the resulting aqueous layer for secondary extraction. The organic layers were combined, and washed with aqueous 5 % sodium hydrogencarbonate solution (3.7 L) and then with water. Activated charcoal was added to the organic layer and stirred for 1 hour, and the activated charcoal separated through filtration was washed with tetrahydrofuran. The filtrate contained 1.629 kg of the intended product (90.6 % assay yield). The toluene/tetrahydrofuran solution was concentrated to about 10.0 L under reduced pressure. Isopropyl acetate (9.77 L) was added to the residue, and further concentrated to about 9.77 L. This operation was repeated three times until the residue of tetrahydrofuran and toluene could reach at most 0.3 % and 1.0 % respectively, A crystal gradually deposited in solvent exchange, and this was stirred overnight at 0 to 5°C. By high-performance liquid chromatography, it was confirmed that the concentration of the intended product in the supernatant is 14.5 mg/mL. The crystal was taken out through filtration, and washed with cold isopropyl acetate (1.63 L) and then with n-heptane (4.89 L). The crystal was dried in a nitrogen atmosphere to obtain 1.46 kg of the intended product as a crude yellow crystal.

### 5) Recrystallization:

The crude compound obtained in 4) (1.46 kg) was dissolved in dimethylacetamide (5.84 L) at 50°C. The solution was filtered through a 0.2-µm filter. A seed crystal was added to the solution, and then stirred for 1 hour. Further, a mixture of dimethylacetamide and water (1/1, 13.14 L) that had been filtered through a 0.2-µm filter was dropwise added to it. The resulting slurry was stirred for 2 hours and then cooled to room temperature over 2 hours, and further stirred for 8 hours or more. The crystal was taken out through filtration, and the crystal was washed with a mixture of dimethylacetamide and water (3/2, 4.38 L) and then with water (7.3 L. twice) to obtain a recrystallized product of the compound as an yield of 89 %.

Further, the recrystallized compound (1.33 kg as a pure content) was dissolved in dimethylacetamide (3.99 L). A mixture of dimethylacetamide and water (1/1, 3.99 L) that had been filtered through a 0.2-µml filter was dropwise added to the solution. A seed crystal was added to the solution, and stirred for 1 hour. Further, a mixture of dimethylacetamide and water (1/1, 11.97 L) that had been filtered through a 0.2-µm filter was dropwise added to it over 2 hours. The resulting slurry was stirred for 2 hours and then cooled to room temperature over 2 hours, and further stirred for 8 hours or more. The crystal was taken out through filtration, and the crystal was washed with a mixture of dimethylacetamide and water (3/2,3.99 L) and then with water (6.55 L, twice). Thus, the entitled compound was obtained as a twice-recrystallized crystal, as an yield of 89.6 %.
m.p.: 144°C
¹H-NMR (500 MHz, DMSO-d₆, δ ppm): 1.58-1.67 (m, 4H), 1.74-1.82 (2H), 1.96-2.09 (6H), 2.14 (s, 3H), 2.60-2.74 (3H), 4.43-4.46 (m, 1H), 7.09-7.12 (m, 2H), 7.32-7.34 (m, 2H), 7.91-7.97 (m, 3H).

### INDUSTRIAL APPLICABILITY

The invention provides an industrially-excellent process for production of 3-{4-[(1-cyclobutyl-4-piperidinyl)oxy]phenyl}-2-methyl-5-trifluoromethyl-4(3H)-quinazolinone, which is useful as a preventive or a remedy for metabolic system diseases such as obesity, diabetes, hormone secretion disorder, hyperlipemia, gout, fatty liver; circulatory system diseases such as stenocardia, acute/congestive heart failure, myocardial infarction, coronary arteriosclerosis, hypertension, renal disease, electrolyte disorder; and central and peripheral nervous system diseases such as bulimia, emotional disorder, depression, anxiety, delirium, dementia, schizophrenia, attention deficit hyperactivity disorder, memory impairment, Alzheimer disease, Parkinson disease, sleep disorder, cognitive disorder, motion disorder, paresthesia, dysosmia, epilepsy, morphine resistance, narcotic dependence, alcoholism.

## Claims

1. A process for producing 3-{4-[(1-cyclobutyl-4-piperidinyl)oxy]phenyl}-2-methyl-5-trifluoromethyl-4(3H)-quinazolinone, which comprises reacting 2-methyl-5-trifluoromethyl-4H-3,1-benzoxazin-4-one with 4-(1-cyclobutyl-4-piperidinyl)oxyaniline or its acid-addition salt in the presence of an acid.

2. The production process as claimed in claim 1, wherein the acid is acetic acid.

3. The production process as claimed in claim 2, wherein sodium acetate is further added to the reaction system.

4. The production process as claimed in claim 1, wherein 4-(1-cyclobutyl-4-piperidinyl)oxyaniline or its acid-addition salt is prepared according to the following:
(1) a step of reacting 4-hydroxy-N-tert-butoxycarbonylpiperidine with 4-fluoro-1-nitrobenzene in the presence of a base to produce 4-(4-piperidinyl)oxy-N-tert-butoxycarbonyl-1-nitrobenzene,
(2) a step of deprotecting 4-(4-piperidinyl)oxy-N-tert-butoxycarbonyl-1-nitrobenzene to produce 4-(4-piperidinyl)oxy-1-nitrobenzene,
(3) a step of reacting 4-(4-piperidinyl)oxy-1-nitrobenzene with cyclobutanone in an inert solvent to produce 4-(1-cyclobutyl-4-piperidinyl)oxy-1-nitrobenzene, and
(4) a step of reducing 4-(1-cyclobutyl-4-piperidinyl)oxy-1-nitrobenzene in an inert solvent.

5. The production process as claimed in claim 1 , wherein 4-(1-cyclobutyl-4-piperidinyl)oxyaniline or its acid-addition salt is prepared according to the following:
(1) a step of reacting 4-hydroxypiperidine with cyclobutanone in an inert solvent to produce 1-cyclobutyl-4-hydroxypiperidine,
(2) a step of reacting 1-cyclobutyl-4-hydroxypiperidine with 4-fluoro-1-nitrobenzene in the presence of a base to produce 1-cyclobutyl-4-(4-nitro-phenoxy)-piperidine, and
(3) a step of reducing 1-cyclobutyl-4-(4-nitro-phenoxy)-piperidine in an inert solvent.

6. The production process as claimed in claim 1, wherein 2-methyl-5-trifluoromethyl-4H-3.1-benzoxazin-4-one is prepared by reacting 2-amino-6-trifluoromethylbenzoic acid or its salt with acetic anhydride in an inert solvent.

7. The production process as claimed in claim 1, wherein, the acid-addition salt of 4-(1-cyclobutyl-4-piperidinyl)oxyaniline is 4-(4-cyclobutyl-4-piperidinyl)oxyaniline p-toluenesulfonate.
